# FASCICULE DE BREVET EUROPEEN

(11) **EP 0 350 341 B1**
(45) Date de publication et mention de la délivrance du brevet: **10.05.1995**
(21) Numéro de dépôt: 89401347.3
(22) Date de dépôt: 16.05.1989
(51) Int. Cl.: C12N 15/76, C12N 1/21, C12P 21/00

(54) **Vecteurs de clonage et d'expression dans une souche d'actinomycète, procédé de transformation de cette souche, souche d'actinomycète obtenue et préparation de protéines**
Klonier- und Expressionvektoren in einem Aktinomycetenstamm,Verfahren zur Transformation dises Stammes,erhaltener Stamm und Herstellung von Proteinen
Cloning and expression vectors in an Aktinomyces strain,method of transformation of this strain,obtained strain and preparation of proteins

(30) Priorité: 18.05.1988 FR 8806638
(43) Date de publication de la demande: 10.01.1990
(73) Titulaire: CENTRE NATIONAL DE LA RECHERCHE SCIENTIFIQUE (CNRS), 75794 Paris Cédex 16 (FR)
(72) Inventeur: Smokvina, Tamara, F-75005 Paris (FR); Boccard, Frédéric, F-75005 Paris (FR); Guerineau, Michel, F-75006 Paris (FR)
(74) Mandataire: Warcoin, Jacques

(56) Documents cités:
- EP-A- 0 191 643
- EP-A- 0 235 112
- EP-A- 0 243 856
- GENE, vol. 59, 1987, pages 137-144, Amsterdam, NL; J.M. Simonet et al.: "Excision and integration of a self-transmissible replicon of Streptomyces ambofaciens"
- CHEMICALS ABSTRACTS, vol. 105, no. 5, 4 août 1986, pages 154,155, abrégé no. 3644Ou, Columbus, Ohio, USA; Ch. A. OMER et al.: "Structural analysis of plasmid and chromosomal loci involved in site-specific excision and integration of the SLP1 element of Streptomyces coelicolor" & J. Bacteriol. 1986, vol. 166, no. 3, pages 999-1006
- CHEMICAL ABSTRACTS, vol. 99, no. 24, 12 decembre 1983, page 191, abrégé no. 207354h, Columbus, Ohio, USA; E. KATZ et al.: "Cloning and expression of the tyrosinase gene from Streptomyces antibioticus in Streptomyces lividans" & J. Gen. Microbiol. 1983, vol. 129, no. 9, pages 2703-2714
- GENE, vol. 39, no. 1, 1985, pages 11-16, Amsterdam, NL; F. FOOR et al.: "Isolation and characterization of the Streptomyces cattleya temperate phage TG1"
- MOL GEN GENET, vol. 198, no. 1, 1984, pages 35-41, Axel Springer Verlag, Berlin, WG; J.-L. PERNODET et al.: "Plasmids in different strains of Streptomyces ambofaciens: free and integrated form of plasmid pSAM2"
- GENE, vol. 32, 1984, pages 21-30, Amsterdam, NL; G. GRAY et al.: "Synthesis of bovine growth hormone by Streptomyces lividans"

## Description

La présente invention concerne des vecteurs de clonage et d'expression de protéines dans les Actinomycètes ainsi que les souches transformées par ces vecteurs.

Les bactéries appartenant à l'ordre des Actinomycétales, et en particulier celles du genre Streptomyces, occupent une place particulièrement importante en microbiologie industrielle. En effet, depuis la découverte par Waksman d'une souche de Streptomyces produisant la streptomycine, de nombreuses autres souches de Streptomyces productrices d'antibiotiques ont été isolées et exploitées industriellement pendant les quarante dernières années. La richesse et la diversité des métabolites secondaires produits par les Streptomyces sont illustrées par le fait que ceux-ci synthétisent 55 % des 4 973 antibiotiques naturels recensés en 1978. Environ 70 % des antibiotiques produits industriellement le sont par les Streptomyces. Les métabolites secondaires d'origine microbienne ont surtout été utilisés pour leur activité antibactérienne, antifongique ou antitumorale. Des substances possédant ces types d'activité ont été découvertes en très grand nombre pendant la période 1950-1970. Actuellement, le rythme des découvertes pour ces types d'application ralentit mais toute la richesse des métabolites secondaires microbiens est loin d'avoir été exploitée. En effet, de nouveaux domaines d'application sont actuellement explorés, ils concernent les activités antiparasitaires, insecticides, herbicides ou pharmacologiques de ces produits. Dans ces domaines également les produits de Streptomyces sont nombreux : on peut citer la monensine qui domaine le marché des produits contre la coccidiose, les avermectines très actives contre les nématodes parasites du bétail et possédant également une activité insecticide, les nikkomycines bloquant la synthèse de chitine et possédant donc une activité insecticide. Les Streptomyces produisent également des substances intéressantes pour leur activité pharmacologique telles que les anti-inflammatoires et les vasodilatateurs.

Une autre raison de s'intéresser aux Streptomyces est leur utilisation comme hôte pour l'expression de gènes hétérologues. En effet, de nombreux industriels possèdent le savoir-faire et les équipements nécessaires pour cultiver les Streptomyces et exploiter ces cultures. Une culture en phase stationnaire peut rester métaboliquement active pendant plusieurs semaines et continuer à synthétiser des protéines et des métabolites secondaires. Etant donné que les Streptomyces produisent un grand nombre d'enzymes extracellulaires, dont plusieurs sont utilisées industriellement telles que les protéases ou la glucose-isomérase, leur système d'excrétion pourrait être utilisé pour la production de protéines hétérologues. Streptomyces lividans a d'ores et déjà été employé avec succès comme hôte pour l'expression de l'hormone de croissance bovine par la société Biogen (Gray et coll., 1985).

L'utilisation de la souche Streptomyces ambofaciens est particulièrement prisée pour la production de spiramycine, antibiotique de la famille des macrolides produit par Rhône-Poulenc. De plus, il s'agit d'une souche qui semble ne pas présenter de phénomène de restriction vis-a-vis de l'ADN étranger (Cox et coll., 1984), ce qui facilite les expériences de clonage moléculaire. Les principales équipes travaillant sur les Streptomyces producteurs de macrolides et utilisant donc S. ambofaciens sont celle du Pr. Omura au Japon, celle de R.H. Baltz de la société Eli Lilly aux Etats Unis et celle du Dr. A. Sabatier de la société Rhône Poulenc Santé.

L'équipe de S. Omura s'intéresse principalement aux voies de biosynthèse des macrolides, à la découverte de nouveaux macrolides et à l'obtention de macrolides hybrides (Omura, 1984). L'équipe de Baltz travaille surtout avec S. fradiae et S. erythraeus produisant respectivement la tylosine et l'érythromycine (Baltz et coll., 1983), mais utilise S. ambofaciens comme hôte dans de nombreuses expériences de clonage moléculaire (Kuhstoss et Rao, 1983 ; Matsuhima et Baltz, 1985).

C'est suite à l'étude de plusieurs souches de Streptomyces ambofaciens appartenant à deux isolats d'origine géographique différente que la présence d'un plasmide particulier a été détecté : le plasmide pSAM2 (Mol. Gen. Genet. (1984) 198:35-41, "Plasmids in different strains of Streptomyces ambofaciens : free and integrated form of plasmid pSAM2" ; et Gene, 59 (1987) 137-144 "Excision and integration of a self-transmissible replicon of Streptomyces ambofaciens").

Le plasmide pSAM2 a une taille de 11 kb, peut exister à l'état libre et/ou intégré et s'est révélé capable de se transférer d'une souche à l'autre. Le phénomène d'intégration du plasmide pSAM2 dans le chromosome des souches réceptrices a aussi pu être mis en évidence mais ce phénomène n'a pas été élucidé à l'heure actuelle.

C'est pourquoi, la présente invention concerne un vecteur de clonage et d'expression d'une séquence d'ADN codant pour une protéine déterminée dans une souche d'Actinomycète, caractérisé en ce qu'il comporte :
. une séquence d'attachement att,
. une séquence d'ADN codant pour une séquence int fonctionnelle dans ladite souche, et
. une séquence d'ADN codant pour ladite protéine spécifique et pour les éléments assurant l'expression de ladite séquence dans ladite souche.

Ce vecteur est plus particulièrement destiné à être un vecteur d'expression codant pour une protéine présentant un intérêt par elle-même ou bien favorisant l'obtention de métabolites secondaires tels que ceux décrits précédemment, en particulier les antibactériens, les antifongiques, les antitumoraux, et des protéines telles que les enzymes, qui sont déjà obtenus à partir de souche d'Actinomycètes ou qui peuvent être produits par d'autres organismes.

Parmi les séquences att, il faut citer plus particulièrement les séquences choisies parmi :
. la séquence
   TTCTCTGTCGGGGTGGCGGGATTTGAACCCACGACCTCTTCGTCCCGAA,
. la séquence présentant au moins 50 % d'homologie avec cette séquence.

En effet, l'invention a mis en évidence que le mécanisme d'intégration chromosomique du plasmide pSAM2 implique une séquence d'attachement caractérisée par ce type de séquence nucléotidique.

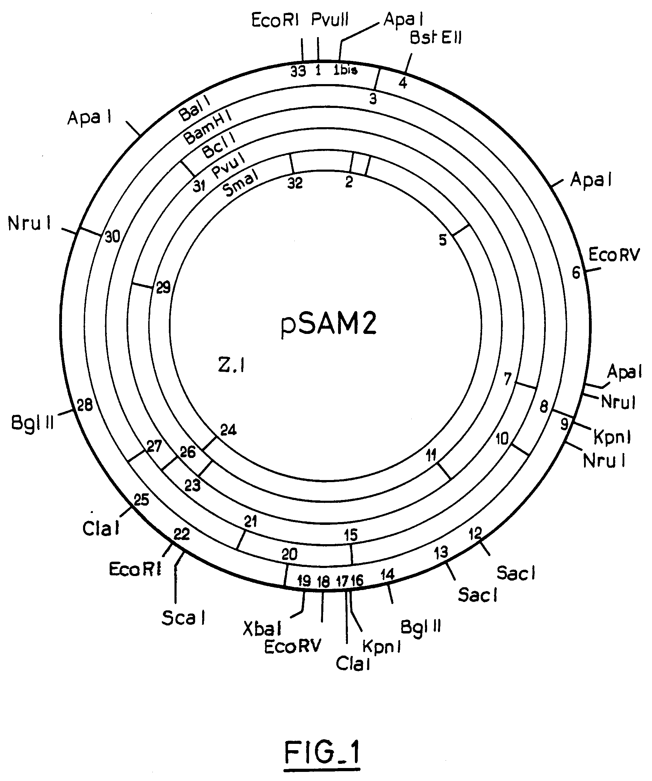

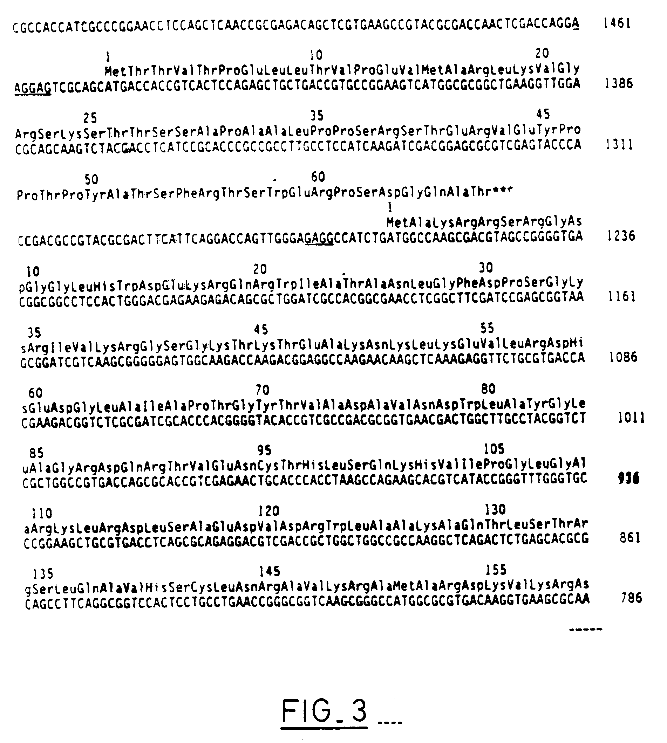

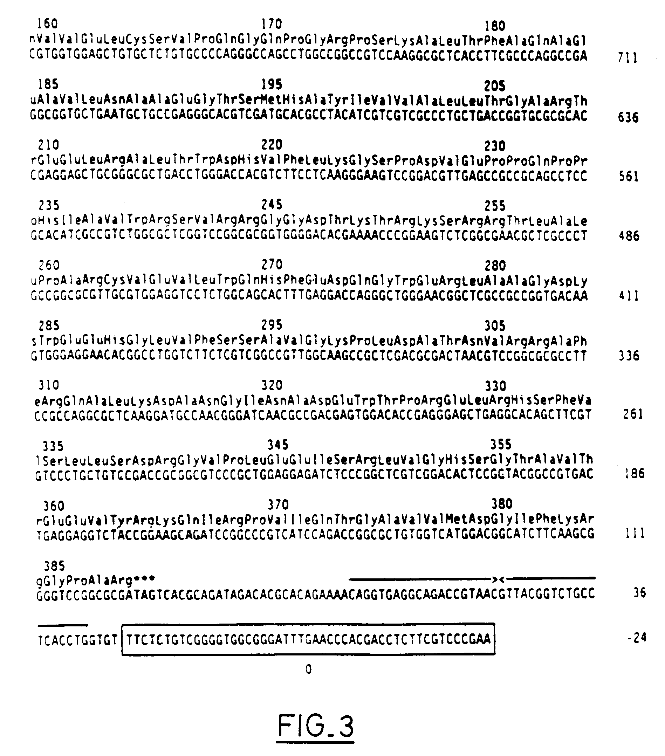

La carte de restriction de pSAM2 a été déterminée et est représentée sur la figure 1. La séquence d'attachement est encadrée sur la figure 3 ; elle débute à la position + 24 et se termine à la position - 24. Le nucléotide en position 0 correspond au centre du site d'attachement.

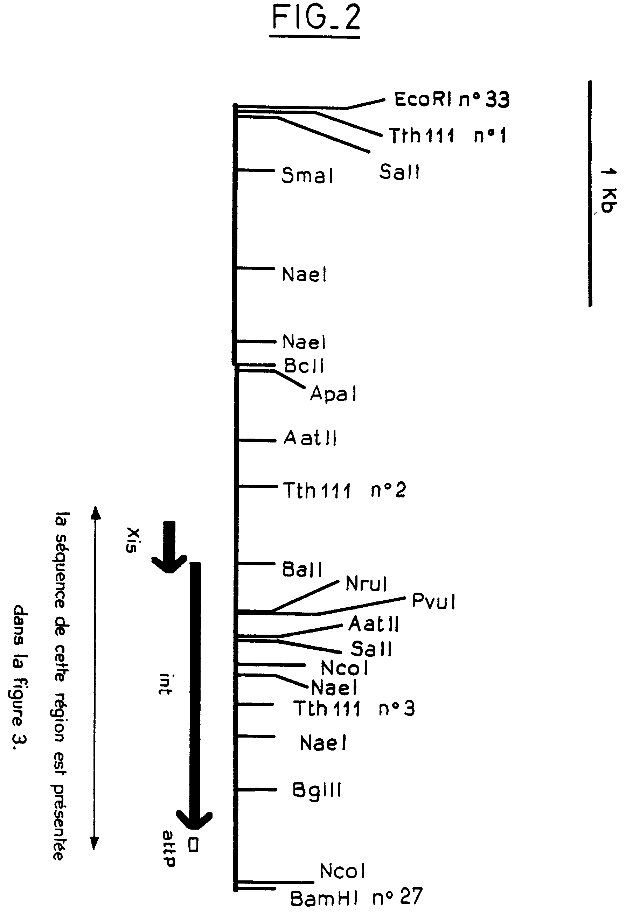

Les vecteurs selon l'invention comportent, en outre, une séquence codant pour une séquence int fonctionnelle dans ladite souche, il s'agit d'une séquence codant pour une intégrase, laquelle séquence précède en général ou est située au voisinage de la séquence d'attachement (voir la figure 2). La séquence int joue un rôle important dans le phénomène d'intégration.

La séquence nucléotidique correspondant à ce gène int a été déterminée et est représentée sur la figure 3 : elle correspond à une séquence d'ADN comprise entre les positions + 1261 et + 97. Cette séquence int est la plupart du temps précédée, ou partiellement chevauchée, par le gène xis qui est le gène codant pour l'excision. La séquence de ce gène est représentée également sur la figure 3.

Les vecteurs selon la présente invention présentent pour la plupart la propriété de s'intégrer dans les chromosomes avec une très grande stabilité.

Afin d'obtenir un grand nombre de copies de vecteur par génome sous forme plasmidique, autoréplicable, avec en plus une copie intégrée ou non intégrée dans le chromosome, il est intéressant d'avoir recours à des vecteurs dans lesquels un fragment de 600 bp a été délété de part et d'autre du site EcoRI n° 33 de pSAM2, c'est-à-dire délété pour l'essentiel d'un fragment qui s'étend entre deux séquences répétées situées de part et d'autre du site EcoRI n° 33 (voir figure 1). De plus, ces vecteurs peuvent contenir les fragments portant les gènes int et xis.

Les vecteurs ainsi obtenus présentent la propriété de se multiplier en assez grand nombre.

Comme tous les vecteurs de clonage ou d'expression, on insérera de préférence les éléments provenant de bactéries, en particulier de E. coli, afin de permettre la construction des vecteurs dans ces bactéries, mais lors de la mise en oeuvre dans les souches d'Actinomycètes, ces fragments correspondants aux bactéries E. coli pourront être supprimés. Les éléments utilisables dans les constructions bactériennes sont connus, il s'agit la plupart du temps d'une origine de réplication permettant de multiplier les plasmides, éventuellement d'une résistance fonctionnelle dans lesdits plasmides et, si nécessaire, d'autres éléments facilitant le montage dans E. coli.

La séquence d'ADN codant pour ladite protéine spécifique et pour les éléments assurant l'expression de ladite séquence dans ladite souche peut être insérée à différents endroits dans le vecteur.

Lorsque l'on souhaite préparer une souche d'Actinomycète produisant une protéine déterminée dont la séquence d'ADN est intégrée dans le chromosome, on utilisera de préférence un vecteur tel que décrit précédemment et dans lequel la séquence d'ADN en cause est intégrée à l'extérieur des séquences att et int, dans ce cas on favorisera l'intégration dans le chromosome.

Lorsque, au contraire, on souhaitera que ce plasmide ne soit pas totalement intégrable dans le chromosome, la séquence d'ADN en cause pourra être insérée dans le gène int.

Dans certains cas où l'on souhaitera que le plasmide vecteur ne puisse pas être transféré d'une souche à l'autre, la séquence d'ADN codant pour la protéine déterminée pourra être insérée au niveau de séquences codant pour les fonctions de transfert fonctionnelles de la souche transformée.

Parmi les séquences codant pour une protéine déterminée, on peut citer les séquences codant pour des résistances à certains types d'antibiotiques, notamment les séquences codant pour une résistance au Thiostrepton.

La résistance au Thiostrepton pourra, dans certains cas, être utilisée comme gène marqueur. Dans ce cas, si l'on souhaite exprimer une autre protéine, il sera nécessaire de prévoir un autre site de clonage, ceci pourra être fait, par exemple, à l'aide d'un vecteur polyvalent en insérant dans l'un des vecteurs décrits précédemment une séquence de "polylinker".

Dans ce dernier cas, le gène marqueur est, de préférence, placé après le site d'attachement dans les vecteurs de l'invention.

Quant à la séquence "polylinker", elle est donc, de préférence, située en aval du gène marqueur, par rapport au site d'attachement le vecteur obtenu étant alors un vecteur intégratif. Un exemple tout particulièrement préféré est le vecteur pTS55 décrit dans l'exemple 1 ci-après.

Parmi les vecteurs intégratifs et réplicatifs obtenus dans le cadre de la présente invention, on peut modifier la position de la séquence "polylinker" de telle sorte que les vecteurs obtenus soient incapables de se transférer d'une souche à l'autre.

De tels vecteurs peuvent être utiles notamment dans le cas de gènes étrangers codant pour des protéines particulièrement avantageuses pour la santé humaine ou animale.

A titre d'exemple, on peut citer le vecteur pTS12 dont la construction sera explicitée dans les exemples ci-après.

La séquence "polylinker" peut aussi être située en amont du gène marqueur, à savoir dans le gène int, de telle sorte que le vecteur obtenu ne puisse pas s'intégrer dans le chromosome des souches réceptrices. A titre d'exemple, on peut citer le vecteur pTS13 dont la construction sera détaillée dans les exemples ci-après.

Les vecteurs de la présente invention sont particulièrement avantageux en raison de leur stabilité et de leur intégration spécifique à large spectre d'hôte.

Leur stabilité permet d'éviter de recourir à une quelconque pression de sélection pour maintenir le gène à l'état intégré.

Dans le cas des vecteurs réplicatifs, on peut penser que les vecteurs également intégratifs sont les plus stables, la copie intégrée pouvant fournir en quelque sorte une matrice permettant de régénérer les formes plasmidiques libres.

Les vecteurs selon l'invention peuvent, en outre, s'intégrer dans toutes les souches possédant le site d'intégration précédemment défini, à savoir un site ayant une séquence comprenant en totalité ou en partie la séquence nucléotidique de la position + 24 à - 24 décrite dans la figure 3. Ce site a été détecté par hybridation avec une sonde allant de la position + 21 à - 18 dans S. antibioticus (DSM 40868), S. bikiniensis (ATCC 11062), S. parvulus (ATCC 12434), S. glaucescens (ETH 22794), S. actuosus (ATCC 25421), S. coelicolor (A3(2)), S. ambofaciens, S. lividans TK64 (66) et S. griseofuscus (DSM 40191).

Pour cette dernière souche, on s'est aperçu que le quatrième nucléotide, à savoir le nucléotide en position + 21, n'était pas une thymidine (T) mais une adénine (A).

Un tel remplacement n'affecte pas la possibilité d'une intégration avec les vecteurs de la présente invention.

De façon générale, au moins 50 % des nucléotides doivent demeurer inchangés pour que la possibilité d'intégration ne soit pas affectée.

La présente invention concerne aussi le procédé de préparation d'une souche d'Actinomycète produisant une protéine déterminée dont la séquence d'ADN est :
. soit intégrée dans le chromosome, la séquence d'ADN en cause étant insérée à l'extérieur des séquences att et int ; de préférence, cette séquence d'ADN est insérée dans les séquences codant pour les fonctions de transfert ;
. soit intégrée dans un plasmide non intégrable totalement dans le chromosome, caractérisé en ce que la séquence d'ADN en cause est intégrée dans le gène int.

La présente invention s'étend aux souches d'Actinomycète, en particulier les Streptomyces, transformées par les vecteurs de la présente invention. La présente invention concerne aussi l'utilisation industrielle de ces souches transformées, et ce, notamment, pour l'obtention d'une protéine déterminée, par fermentation d'une souche transformée selon la présente invention dans un milieu de culture approprié et récupération de la protéine déterminée.

De très nombreuses protéines peuvent être préparées grâce aux vecteurs de la présente invention.

Ainsi, certains vecteurs seront plus particulièrement adaptés à des protéines dont on ne souhaite pas une expression forte. Il s'agit, notamment, du vecteur pTS55 qui permet l'intégration d'un seul gène dans le chromosome. Il y a donc une copie unique du plasmide par cellule transformée. Un tel vecteur convient à l'expression de protéines enzymatiques telles que l'amylase ou des gènes de régulation agissant sur la production de protéines ou de métabolites industriellement intéressants.

D'autres vecteurs seront plutôt appropriés à l'expression de protéines étrangères dont on exige une expression importante. C'est ainsi que les vecteurs pTS13 et pTS12, du fait de la délétion de part et d'autre du site EcoRI n° 33 de pSAM2 (voir les exemples suivants), se maintiennent à un taux très élevé de copies lorsqu'ils sont introduits dans Streptomyces et notamment Streptomyces lividans TK64. Le nombre de copies est supérieur à 100 par génome.

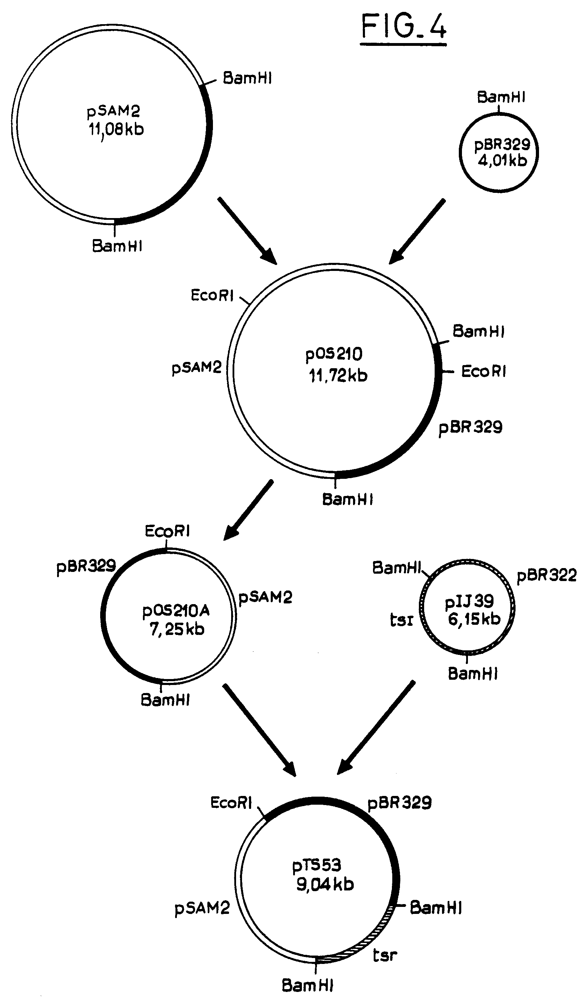

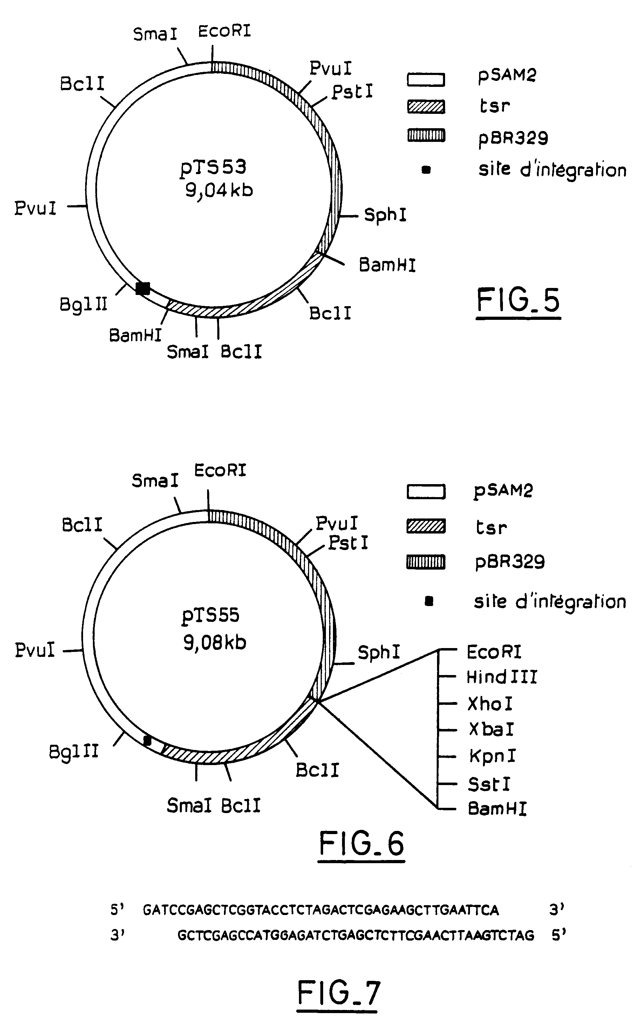

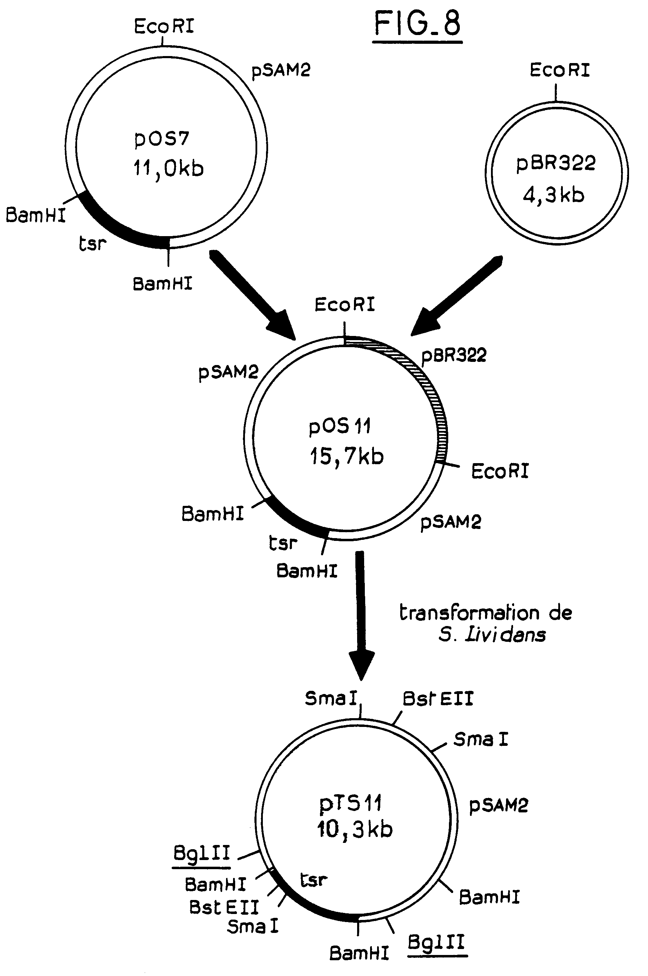

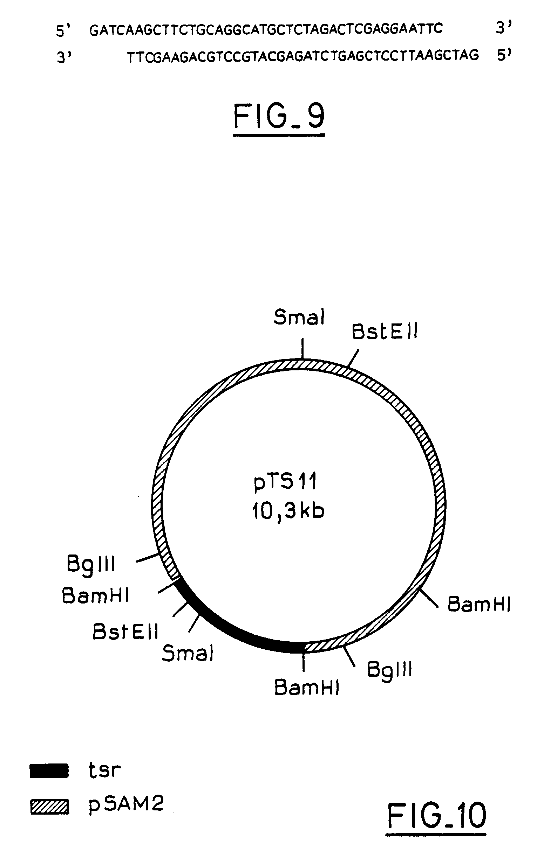

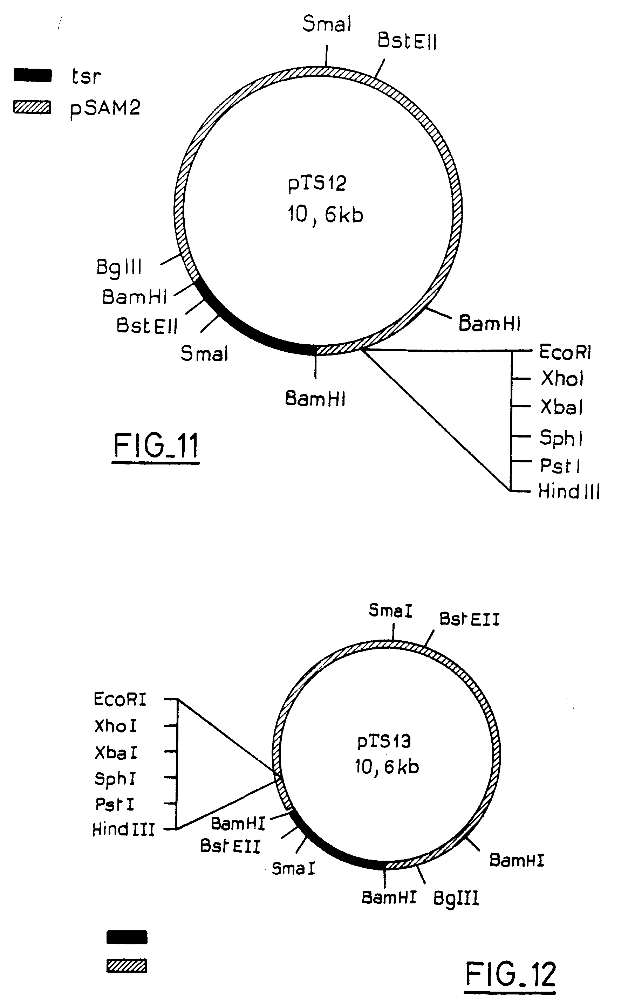

La présente invention est illustrée à l'aide des exemples et des figures suivants :
. la figure 1 représente la carte de restriction du plasmide pSAM2 : les sites de restriction sont numérotés de 1 à 33, le premier site est PvuII (n° 1), le dernier est EcoRI (n° 33) ;
. la figure 2 représente la carte de restriction du fragment BamHI-EcoRI de pSAM2 suffisant pour l'intégration, ce fragment va du site BamHI n° 27 au site EcoRI n° 33 ;
. la figure 3 représente la séquence de la région comprise entre Tth111 n° 2 et le site d'intégration att de pSAM2 dont la carte de restriction est représentée sur la figure 2, il s'agit de la séquence nucléotidique du plasmide pSAM2, cette séquence a été déterminée à partir de 70 nucléotides après le site Tth111 n° 2 jusqu'au site d'intégration (att), le nucléotide 0 correspond au milieu de att ;
. la figure 4 schématise la construction de pTS53 ;
. la figure 5 représente la carte de restriction du vecteur pTS53 ;
. la figure 6 représente la carte de restriction du vecteur intégratif pTS55 ;
. la figure 7 représente la séquence de l'oligonucléotide introduit dans pTS54 afin de construire pTS55 ;
. la figure 8 schématise la construction de pTS11 ;
. la figure 9 représente la séquence de l'oligonucléotide introduit dans pTS11 afin de construire pTS12 et pTS13 ;
. la figure 10 représente la carte de restriction de pTS11 ;
. la figure 11 représente la carte de restriction du plasmide multicopie pTS12 ;
. la figure 12 représente la carte de restriction du plasmide pTS13.

### EXEMPLE 1 : Construction du vecteur intégratif pTS55

Le plasmide pSAM2 (figure 1) a été extrait et purifié de Streptomyces ambofaciens (JI3212) selon la méthode de lyse alcaline décrite par Hopwood dans "Genetic Manipulation of Streptomyces" (A Laboratory Manual, 1985).

### 1ère étape :

Le plasmide pSAM2 a été digéré par l'enzyme de restriction BamHI et un fragment de 7,57 kb (kb : kilobase) a été isolé et cloné au site BamHI du plasmide pBR329, ce qui donne le plasmide pOS210 (figure 4).

### 2ème étape :

Le plasmide pOS210 a été digéré par EcoRI et le fragment de 7,25 kb qui contient le fragment de pSAM2 du site BamHI n° 27 au site EcoRI n° 33 et le fragment BamHI-EcoRI de 3,55 kb du plasmide pBR329 a été isolé et refermé sur lui-même, ce qui donne le plasmide pOS210A (figure 4).

### 3ème étape :

Le plasmide PIJ39 correspond au plasmide pBR322 qui contient le gène conférant la résistance au thiostrepton porté par un fragment BamHI de 1,8 kb de Streptomyces azureus (Thompson C.J. ; Ward J.M. ; Hopwood D.A., Nature 286, 525-527, 1980).

Le plasmide pIJ39 est digéré par BamHI et le fragment de 1,8 kb portant la résistance au thiostrepton est isolé et mélangé avec le plasmide pOS210A digéré par BamHI (BamHI est un site unique dans pOS210A).

### 4ème étape :

Le plasmide pTS52 contient deux sites BamHI et a une taille de 9,08 kb. Il est digéré partiellement par BamHI et le plasmide linéarisé de 9,08 kb est isolé. Il est traité par le fragment de Klenow de la polymérase I puis refermé sur lui-même pour éliminer un des deux sites BamHI.

Le plasmide pTS54 portant un seul site BamHI localisé entre les sites SphI et BclI est obtenu (figure 5).

### 5ème étape :

L'oligonucléotide double brin polylinker ayant la séquence décrite dans la figure 7 est synthétisé avec l'appareil "Applied biosystem".

Le plasmide pTS54 digéré par BamHI est mélangé avec l'oligonucléotide polylinker et ligaturé grâce à la ligase du phage T4.

Le plasmide résultant pTS55 est décrit dans la figure 6. Il possède les sites uniques de restriction suivants, disponibles pour les clonages : XbaI, XhoI, HindIII, KpnI, SacI, BamHI et SphI.

Le vecteur pTS55 peut être maintenu dans E. coli en sélectionnant pour la résistance à l'ampicilline.

Par digestion par l'enzyme de restriction EcoRI la partie pBR329 peut être retirée car elle n'est pas indispensable pour l'intégration dans le chromosome des Streptomyces. On peut donc obtenir des transformants intégratifs de Streptomyces qui ne contiennent que de l'ADN de Streptomyces.

Dans les transformants Streptomyces, il confère la résistance à l'antibiotique Thiostrepton ou Nosiheptide.

Il porte le fragment BamHI-EcoRI de pSAM2 et la séquence nucléotidique du site Tth111 n° 2 au site d'intégration du plasmide localisé entre BglII et BamHI a été réalisée (voir figure 2) et est décrite dans la figure 3.

La phase codante qui part de la position + 1261 et se termine à la position + 97 correspond à l'intégrase requise pour l'intégration du plasmide dans le chromosome. La phase codante qui part de la position + 1448 et se termine à la position + 1247 peut correspondre au gène xis.

La séquence encadrée qui part de la position + 24 à la position - 24 correspond au site d'intégration du plasmide (att). Le nucléotide position 0 correspond au centre du site d'attachement. Il peut être introduit par transformation dans Streptomyces selon la technique décrite dans le "Laboratory Manual". Il s'intègre spécifiquement dans les souches suivantes :
- S. ambofaciens,
- S. lividans TK64,
- S. griseofuscus.

Il peut s'intégrer dans toutes les souches possèdant un site d'intégration pour le plasmide. Ce site peut être defini comme ayant une séquence similaire ou comprenant en totalité ou en partie la séquence nucléotidique de la position + 24 à - 24 décrite dans la figure 3. Ce site a été détecté par hybridation avec une sonde allant de la position + 21 à - 18 (figure 3) dans S. antibioticus, S. bikiniensis, S. parvulus, S. actuosus, S. coelicolor, S. glaucescens.

Ce vecteur permet l'intégration d'un gène dans le chromosome à un site spécifique. Il n'y a donc qu'une copie par cellule, l'intégration est très stable et il n'est donc pas nécessaire de maintenir une pression de sélection pour que le gène introduit reste intégre.

### EXEMPLE 2 : Construction et propriétés du vecteur libre pTS39

### a) construction

Le plasmide pSAM2 originaire de S. ambofaciens JI3212 est digéré partiellement par EcoRI et le plasmide linéarisé de 11 kb est isolé et cloné dans le site EcoRI du plasmide PIJ39 (précédemment décrit). Le plasmide résultant pTS39 contient le plasmide pIJ39 cloné au site EcoRI N° 22 du plasmide pSAM2 (carte de restriction pSAM2, figure 1).

### b) propriétés

Le plasmide peut être introduit par transformation dans différentes souches de Streptomyces où il se maintient à l'état libre et intégré en conférant la résistance au Thiostrepton et au Nosiheptide. C'est un vecteur à large spectre d'hôte. Le nombre de copies est compris entre 5 et 20 copies par génome.

### EXEMPLE 3 : Construction et propriétés du vecteur pOS11

### 1ère étape :

Le plasmide pOS7 a été décrit dans l'article de J.M. Simonet et al., Gene 59, p. 137-144, 1987. Les deux fragments BamHI de 0,88 kb (BamHI N° 15 à 21) et de 1,01 kb (BamHI N° 21 à 27) de pSAM2 (figure 1) ont été délétés et remplacés par le fragment BamHI de 1,79 kb de S. azureus conférant la résistance au Thiostrepton et au Nosiheptide.

Le plasmide pOS7 et le plasmide pBR322 ont été digérés par EcoRI, mélangés et ligaturés. Le plasmide résultant, pOS11, est obtenu. Il contient pBR322 cloné au site EcoRI N° 33 de pSAM2 (voir figure 8).

### 2ème étape :

Par transformation selon le procédé habituel, il est introduit dans S. lividans où il confère la résistance au Thiostrepton et au Nosiheptide.

D'un clone de S. lividans transformant on a extrait le plasmide pTS11 (figure 8).

Ce plasmide ne contient plus le plasmide pBR322 et a subi une délétion de 600 paires de bases situées de part et d'autre du site EcoRI N° 33 de pSAM2. Le site PvuII N° 1 et le site ApaI N° 1 bis ont disparu (voir carte de restriction pSAM2, figure 1).

### 3ème étape :

Le plasmide pTS11 (figure 10) est digéré partiellement par BglII afin de linéariser le plasmide qui a une taille de 10,3 kb. Les molécules linéaires ayant la taille du plasmide sont isolées.

L'oligonucléotide polylinker ayant la séquence décrite dans la figure 10 est synthétisé.

Il est mélangé au fragment BglII de pTS11 et ligaturé. Le plasmide résultant qui porte l'oligonucléotide inséré au site BglII N° 14 est appelé pTS12 (figure 11) et celui qui porte l'oligonucléotide inséré au site BglII N° 28 est appelé pTS13 (figure 12).

De la même manière il serait possible d'insérer un polylinker aux sites BamHI bordant le fragment BamHI de 1,79 kb portant le gène de résistance au Thiostrepton.

Les propriétés des vecteurs obtenus sont les suivantes :

Ces vecteurs grâce à la délétion de part et d'autre du site EcoRI N° 33 de pSAM2 se maintiennent à un taux très élevé de copies lorsqu'ils sont introduits dans Streptomyces et notamment Streptomyces lividans TK64. Le nombre de copies est supérieur à 100 par génome.

Ils confèrent la résistance au Thiostrepton et portent les sites de clonage HindIII, PstI, SphI, XbaI, XhoI et EcoRI.

Le vecteur pTS13 est incapable de s'intégrer dans le chromosome et est uniquement autoréplicatif.

Le vecteur pTS12 ne devrait plus être capable de se transférer et ne serait plus conjugatif.

Tout vecteur dérivé de pSAM2 contenant cette délétion passe de 10 copies environ à plus de 100 copies.

### EXEMPLE 4 : Expression d'un gène d'alpha-amylase de Streptomyces limosus en utilisant un dérivé intégratif du plasmide pSAM2 Procédé de construction

L'interposon Omega conférant la résistance à l'antibiotique spectinomycine (Prentki et Krish, 1984, Gene 29, p. 303-312) a été inséré au site EcoRI du plasmide pOS210 A de la figure 4. Un fragment portant le gène de structure de l'alpha-amylase de S. limosus ainsi que les séquences promotrices (Long et at., 1987, Journal of bacteriology 169, p. 5745-5754) (ce gène a été breveté par la société Cetus) ont été insérés au site BamHI du plasmide résultant. Ce plasmide recombinant a été introduit par transformation dans S. lividans. Les transformants sélectionnés pour la résistance à l'antibiotique spectinomycine sont très stables et produisent de l'alpha-amylase sans pression de sélection pour maintenir le plasmide recombinant intégré.

### BIBLIOGRAPHIE

Batlz R.H., Seno E.T., Stonesifer J. and Wild G.M. (1983) Biosynthesis of the macrolide antibiotic tylosin a preferred pathway from tylactone to tylosin. J. of Antibiotics 36 n° 2 131-141.

Cox K.L., Baltz R.H. (1984) Restriction of Bacteriophage plaque formation in Streptomyces ssp. J. Bact. 159.

Gray G., Seltzer G., Buell G., Shaw P., Escanez S., Hofer S., Voegeli P. and Thompson C.J. (1984) Synthesis of bovine growth by Streptomyces lividans. Gene 1124, 21-30.

Kuhstoss S., and Nagaraja Rao R. (1983) Expression in Streptomyces ambofaciens of an Escherichia coli K-12 gene which confers resistance to hygromycin B. Gene 26, 295-299.
Matsushima P., Baltz R.H. (1985) Efficient plasmid transformation of Streptomyces ambofaciens and Streptomyces fradiae protoplasts. J. Bact. 163, 180-185.

Omura S. (1984) Macrolide Antibiotics : Chemistry, Biology and Practice. Academic Press.

Covarrubias L., Bolivar F. (1982) Construction and characterization of new cloning vehicles. VI. Plasmid pBR329, a new derivative of pBR328 lacking the 482 base-paire inverted duplication. Gene 17 : 79-89.

## Revendications (Revendications pour l'(les) Etat(s) contractant(s) suivant(s): AT, BE, CH, LI, DE, FR, GB, IT, LU, NL, SE)

1. Vecteur de clonage et d'expression d'une séquence d'ADN codant pour une protéine déterminée dans une souche d'Actinomycète, caractérisé en ce qu'il consiste essentiellement en :
. une séquence d'attachement att,
. une séquence d'ADN codant pour une intégrase (séquence int) fonctionnelle dans ladite souche et,
. une séquence d'ADN codant pour ladite protéine spécifique et pour les éléments assurant l'expression de ladite séquence dans ladite souche.

2. Vecteur selon la revendication 1, caractérisé en ce que la séquence att est choisie parmi :
. la séquence TTCTCTGTCGGGGTGGCGGGATTTGAACCCACGACCTCTTCGTCCCGAA
. la séquence présentant au moins 50 % d'homologie avec cette séquence.

3. Vecteur selon la revendication 2, caractérisé en ce que la séquence att est choisie parmi celles qui s'hybrident dans S.antibioticus (DSM 40868), S. bikiniensis (ATCC 11062), S. Parvulus (ATCC 12434), S. glaucescens (ETH22794), S. actuosus (ATCC25421), S. coelicolor (A3(2)), S. ambofaciens, S. lividans TK64(66), et S.griseofuscus (DSM40191).

4. Vecteur selon l'une des revendications 1 à 3, caractérisé en ce que les fragments att et int proviennent d'un fragment de pSAM2, qui a été délété d'environ 600 bp de part et d'autre du site EcoRI n° 33.

5. Vecteur selon la revendication 4, caractérisé en ce que la délétion porte sur un fragment qui s'étend entre deux séquences répétées.

6. Vecteur selon l'une des revendications 1 à 5, caractérisé en ce qu'il comporte, en outre, les fonctions de transfert fonctionnelles dans ladite souche.

7. Procédé de préparation d'une souche d'Actinomycète produisant une protéine déterminée dont la séquence d'ADN est intégrée dans le chromosome, caractérisé en ce qu'on transforme la souche à l'aide d'un vecteur selon l'une des revendications 1 à 6, dans lequel la séquence d'ADN en cause est insérée à l'extérieur des séquences att et int.

8. Procédé selon la revendication 7, caractérisé en ce que le vecteur est non transférable, la séquence d'ADN codant pour ladite protéine étant insérée dans les séquences codant pour les fonctions de transfert.

9. Procédé de préparation d'une souche d'Actinomycète produisant une protéine déterminée dont la séquence d'ADN est intégrée dans un vecteur selon l'une des revendications 1 à 6 qui est un plasmide non intégrable totalement dans le chromosome, caractérisé en ce que ladite séquence d'ADN est intégrée dans le gène int.

10. Procédé selon l'une des revendications 7 à 9, caractérisé en ce que la séquence codant pour une protéine déterminée est la séquence codant pour la résistance du Thiostrepton.

11. Souche d'Actinomycète obtenue par mise en oeuvre du procédé selon l'une des revendications 7 à 10.

12. Souche selon la revendication 11, caractérisé en ce qu'il s'agit d'une souche de Streptomyces.

13. Procédé d'obtention d'une protéine déterminée, caractérisé en ce qu'on fait fermenter une souche selon l'une des revendications 11 ou 12, dans un milieu de culture approprié et en ce que l'on récupère la protéine déterminée.

14. Séquence d'attachement att telle que définie à l'une des revendications 2 à 5.

15. Séquence int telle que définie aux revendications 4 ou 5.

## Revendications (Revendications pour l'(les) Etat(s) contractant(s) suivant(s): ES, GR)

1. Procédé de préparation d'une souche d'Actinomycète produisant une protéine déterminée dont la séquence d'ADN est intégrée dans le chromosome, caractérisé en ce qu'on transforme la souche a l'aide d'un vecteur de clonage et d'expression de ladite séquence d'ADN consistant essentiellement en :
. une séquence d'attachement att,
. une séquence d'ADN codant pour une intégrase (séquence int) fonctionnelle dans ladite souche et,
. une séquence d'ADN codant pour ladite protéine spécifique et pour les éléments assurant l'expression de ladite séquence dans ladite souche et dans lequel la séquence d'ADN en cause est insérée a l'exterieur des séquences att et int.

2. Procédé selon la revendication 1, caractérisé en ce que le vecteur est non transférable, la séquence d'ADN codant pour ladite protéine étant insérée dans les séquences codant pour les fonctions de transfert.

3. Procédé de préparation d'une souche d'Actinomycéte produisant une protéine déterminée dont la séquence d'ADN est intégrée dans un vecteur de clonage et d'expression de ladite séquence d'ADN consistant essentiellement en :
. une séquence d'attachement att,
. une séquence d'ADN codant pour une intégrase (séquence int) fonctionnelle dans ladite souche et,
. une séquence d'ADN codant pour ladite protéine spécifique et pour les éléments assurant l'expression de ladite sèquence dans ladite souche, ledit vecteur étant un plasmide non intégrable totalement dans le chromosome et ladite séquence d'ADN étant intégrée dans le gène int.

4. Procédé selon l'une des revendications 1 à 3, caractérisé en ce que la séquence att est choisie parmi :
. la séquence TTCTCTGTCGGGGTGGCGGGATTTGAACCCACGACCTCTTCGTCCCGAA
. la séquence présentant au moins 50 % d'homologie avec cette sèquence.

5. Procédé selon la revendication 4, caractérisé en ce que la séquence att est choisie parmi celles qui s'hybrident dans S.antibioticus (DSM 40808), S. bikiniensis (ATCC 11062), S. Parvulus (ATCC 12434), S. glaucescens (ETH 22794), S. actuosus (ATCC 25421), S. coelicolor (A3(2)), S. ambofaciens, S. lividans TK64 (66), et S. griseofuscus (DSM 40191).

6. Procédé selon l'une des revendications 1 a 5, caractérisé en ce que les fragments att et int proviennent d'un fragment de pSAM2, qui a été délété d'environ 600 bp de part et d'autre du site EcoRI n° 33.

7. Procédé selon la revendication 6, caractérisé en ce que la délétion porte sur un fragment qui s'étend entre deux séquences répétées.

8. Procédé selon l'une des revendications 1 à 5, caractérisé en ce qu'il comporte, en outre, les fonctions de transfert fonctionnelles dans ladite souche.

9. Procédé selon l'une des revendications 1 a 8, caractérisé en ce que la séquence codant pour une protéine déterminée est la séquence codant pour la résistance du Thiostrepton.

10. Souche d'Actinomycète obtenue par mise en oeuvre du procédé selon l'une des revendications 1 à 9.

11. Souche selon la revendication 10, caractérisé en ce qu'il s'agit d'une souche de Streptomyces.

12. Procédé d'obtention d'une protéine déterminée, caractérisé en ce qu'on fait fermenter une souche selon l'une des revendications 10 ou 11, dans un milieu de culture approprié et en ce que l'on récupère la protéine déterminée.

## Claims (Claims for the following Contracting State(s): AT, BE, CH, LI, DE, FR, GB, IT, LU, NL, SE)

1. Vector for the cloning and expression of a DNA sequence encoding a given protein in an Actinomycete strain, characterized in that it consists essentially of:
· a sequence for attachment att,
· a DNA sequence encoding a functional integrase (sequence int) in the said strain and,
· a DNA sequence encoding the said specific protein and the elements ensuring the expression of the said sequence in the said strain.

2. Vector according to Claim 1, characterized in that the att sequence is chosen from:
· the sequence TTCTCTGTCGGGGTGGCGGGATTTGAACCCACGACCTCTTCGTCCCGAA
· the sequence having at least 50% homology with this sequence.

3. Vector according to Claim 2, characterized in that the att sequence is chosen from those which hybridize in S. antibioticus (DSM 40868), S. bikiniensis (ATCC 11062), S. Parvulus (ATCC 12434), S. glaucescens (ETH22794), S. actuosus (ATCC 25421), S. coelicolor (A3(2)), S. ambofaciens, S. lividans TK64(66), and S. griseofuscus (DSM 40191).

4. Vector according to one of Claims 1 to 3, characterized in that the att and int fragments are derived from a fragment of pSAM2 which has been deleted of about 600 bp on either side of the EcoRI site No. 33.

5. Vector according to Claim 4, characterized in that the deletion is made on a fragment which extends between two repeat sequences.

6. Vector according to one of Claims 1 to 5, characterized in that it comprises, in addition, transfer functions which are functional in the said strain.

7. Process for the preparation of an Actinomycete strain producing a given protein whose DNA sequence is integrated into the chromosome, characterized in that the strain is transformed by means of a vector according to one of Claims 1 to 6, in which the DNA sequence in question is inserted outside the att and int sequences.

8. Process according to Claim 7, characterized in that the vector is non-transferable, the DNA sequence encoding the said protein being inserted into sequences encoding the transfer functions.

9. Process for the preparation of an Actinomycete strain producing a given protein whose DNA sequence is integrated into a vector according to one of Claims 1 to 6 which is a plasmid which is not completely integrable into the chromosome, characterized in that the said DNA sequence is integrated into the int gene.

10. Process according to one of Claims 7 to 9, characterized in that the sequence encoding a given protein is the sequence encoding the Thiostrepton resistance.

11. Actinomycete strain obtained using the process according to one of Claims 7 to 10.

12. Strain according to Claim 11, characterized in that it is a Streptomyces strain.

13. Process for the production of a given protein, characterized in that a strain according to either of Claims 11 or 12 is fermented in an appropriate culture medium and in that the given protein is recovered.

14. Sequence for attachment att as defined in one of Claims 2 to 5.

15. Sequence int as defined in Claims 4 or 5.

## Claims (Claims for the following Contracting State(s): ES, GR)

1. Process for the preparation of an Actinomycete strain which produces a given protein whose DNA sequence is integrated into the chromosome, characterized in that the strain is transformed by means of a vector for the cloning and expression of the said DNA sequence consisting essentially of:
· a sequence for attachment att,
· a DNA sequence encoding a functional integrase (sequence int) in the said strain and,
· a DNA sequence encoding the said specific protein and the elements ensuring the expression of the said sequence in the said strain and in which the DNA sequence in question is inserted outside the att and int sequences.

2. Process according to Claim 1, characterized in that the vector is non-transferable, the DNA sequence encoding the said protein being inserted into sequences encoding the transfer functions.

3. Process for the preparation of an Actinomycete strain which produces a given protein whose DNA sequence is integrated into a vector for the cloning and expression of the said DNA sequence consisting essentially of:
· a sequence for attachment att,
· a DNA sequence encoding a functional integrase (sequence int) in the said strain and,
· a DNA sequence encoding the said specific protein and the elements ensuring the expression of the said sequence in the said strain, the said vector being a plasmid which is not completely integrable into the chromosome and the said DNA sequence being integrated into the int gene.

4. Process according to one of Claims 1 to 3, characterized in that the att sequence is chosen from:
· the sequence
TTCTCTGTCGGGGTGGCGGGATTTGAACCCACGACCTCTTCGTCCCGAA
· the sequence having at least 50% homology with this sequence.

5. Process according to Claim 4, characterized in that the att sequence chosen from those which hyhridize in S. antibioticus (DSM 40868), S. bikiniensis (ATCC 11062), S. Parvulus (ATCC 12434), S. glaucescens (ETH 22794), S. actuosus (ATCC 25421), S. coelicolor (A3(2)), S. ambofaciens, S. lividans TK64(66), and S. griseofuscus (DSM 40191).

6. Process according to one of Claims 1 to 5, characterized in that the att and int fragments are derived from a fragment pSAM2 which has been deleted of about 600 bp on either side of the EcoRI site No. 33.

7. Process according to Claim 6, characterized in that the deletion is made on a fragment which extends between two repeat sequences.

8. Process according to one of Claims 1 to 5, characterized in that it comprises, in addition, transfer functions which are functional in the said strain.

9. Process according to Claims 1 to 8, characterized in that the sequence encoding a given protein is the sequence encoding the Thiostrepton resistance.

10. Actinomycete strain obtained using the process according to one of Claims 1 to 9.

11. Strain according to Claim 10, characterized in that it is a Streptomyces strain.

12. Process for the production of a given protein, characterized in that a strain according to either of Claims 10 or 11 is fermented in an appropriate culture medium and in that the given protein is recovered.

## Patentansprüche (Patentansprüche für folgende(n) Vertragsstaat(en): AT, BE, CH, LI, DE, FR, GB, IT, LU, NL, SE)

1. Vektor zur Klonierung und Expression einer DNA-Sequenz, die für ein vorgegebenes Protein in einem Actinonyceten-Stamm codiert, dadurch gekennzeichnet, daß er im wesentlichen besteht aus:
- einer Bindungs-Sequenz att,
- einer DNA-Sequenz, die für eine funktionelle Integrase (Sequenz int) in dem genannten Stamm codiert, und
- einer DNA-Sequenz, die für das genannte spezifische Protein und für die Elemente codiert, welche die Expression der genannten Sequenz in dem genannten Stamm gewährleisten.

2. Vektor nach Anspruch 1, dadurch gekennzeichnet, daß die Sequenz att ausgewählt wird aus:
- der Sequenz
TTCTCTGTCGGGGTGGCGGGATTTGAACCCACGACCTCTTCGTCCCGAA und
- der Sequenz, die eine Homologie von mindestens 50 % mit dieser Sequenz aufweist.

3. Vektor nch Anspruch 2, dadurch gekennzeichnet, daß die Sequenz att ausgewählt wird aus solchen, die sich in S. antibioticus (DSM 40868), S. bikiniensis (ATCC 11062), S. parvulus (ATCC 12434), S. glaucescens (ETH22794), S. actuosus (ATCC25421), S. coelicolor (A3(2)), S. ambofaciens, S. lividans TK64(66) und S. griseofuscus (DSM40191) hybridisieren.

4. Vektor nach einem der Ansprüche 1 bis 3, dadurch gekennzeichnet, daß die Fragmente att und int aus einem Fragment von pSAM2 stammen, aus dem etwa 600 bp beiderseits der Stelle EcoRI Nr. 33 deletiert worden sind.

5. Vektor nach Anspruch 4, dadurch gekennzeichnet, daß die Deletion Aufschluß gibt über ein Fragment, das sich zwischen zwei wiederkehrenden Sequenzen erstreckt.

6. Vektor nach einem der Ansprüche 1 bis 5, dadurch gekennzeichnet, daß er außerdem funktionelle Transfer-Funktionen in dem genannten Stamm enthält.

7. Verfahren zur Herstellung eines Actinomyceten-Stammes, der ein vorgegebenes Protein bildet, dessen DNA-Sequenz in das Chromosom integriert ist, dadurch gekennzeichnet, daß man den Stamm mit Hilfe eines Vektors nach einem der Ansprüche 1 bis 6 transformiert, in dem die fragliche DNA-Sequenz außerhalb der Sequenzen att und int inseriert worden ist.

8. Verfahren nach Anspruch 7, dadurch gekennzeichnet, daß der Vektor nicht transferierbar ist, wobei die DNA-Sequenz, die für das genannte Protein codiert, in die Sequenzen inseriert ist, die für die Transferfunktionen codieren.

9. Verfahren zur Herstellung eines Actinomyceten-Stammes, der ein vorgegebenes Protein bildet, dessen DNA-Sequenz in einen Vektor nach einem der Ansprüche 1 bis 6 integriert ist, der ein in das Chromosom nicht vollständig integrierbares Plasmid ist, dadurch gekennzeichnet, daß die genannte DNA-Sequenz in das Gen int integriert ist.

10. Verfahren nach einem der Ansprüche 7 bis 9, dadurch gekennzeichnet, daß die Sequenz, die für ein vorgegebenes Protein codiert, die Sequenz ist, die für die Resistenz gegenüber Thiostrepton codiert.

11. Actinomyceten-Stamm, wie er bei Durchführung des Verfahrens nach einem der Ansprüche 7 bis 10 erhalten wird.

12. Stamm nach Anspruch 11, dadurch gekennzeichnet, daß es sich dabei um einen Streptomyces-Stamm handelt.

13. Verfahren zur Herstellung eines vorgegebenen Proteins, dadurch gekennzeichnet, daß man einen Stamm nach einem der Ansprüche 11 oder 12 in einem geeigneten Kulturmedium fermentieren läßt und daß man das vorgegebene Protein daraus gewinnt (abtrennt).

14. Bindungssequenz att, wie sie in einem der Ansprüche 2 bis 5 definiert ist.

15. Sequenz int, wie sie in den Ansprüchen 4 oder 5 definiert ist.

## Patentansprüche (Patentansprüche für folgende(n) Vertragsstaat(en): ES, GR)

1. Verfahren zur Herstellung eines Actinomyceten-Stammes, der ein vorgegebenes Protein bildet, dessen DNA-Sequenz in das Chromosom integriert ist, dadurch gekennzeichnet, daß man den Stamm mit Hilfe eines Vektors zur Klonierung und Expression der genannten DNA-Sequenz transformiert, der im wesentlichen besteht aus:
- einer Bindungs-Sequenz att,
- einer DNA-Sequenz, die für eine funktionelle Integrase (Sequenz int) in dem genannten Stamm codiert, und
- einer DNA-Sequenz, die für das genannte spezifische Protein und für die Elemente codiert, welche die Expression der genannten Sequenz in dem genannten Stamm gewährleisten,
und in den die fragliche DNA-Sequenz außerhalb der Sequenzen att und int inseriert ist.

2. Verfahren nach Anspruch 1, dadurch gekennzeichnet, daß der Vektor nicht transferierbar ist, wobei die DNA-Sequenz, die für das genannte Protein codiert, in die Sequenzen inseriert ist, die für die Transfer-Funktionen codieren.

3. Verfahren zur Herstellung eines Actinomyceten-Stammes, der ein vorgegebenes Protein bildet, dessen DNA-Sequenz in einen Vektor zur Klonierung und Expression der genannten DNA-Sequenz integriert ist, der im wesentlichen besteht aus:
- einer Bindungs-Sequenz att,
- einer DNA-Sequenz, die für eine funktionelle Integrase (Sequenz int) in dem genannten Stamm codiert, und
- einer DNA-Sequenz, die für das genannte spezifische Protein und für die Elemente codiert, welche die Expression der genannten Sequenz in dem genannten Stamm gewährleisten,
wobei der genannte Vektor ein nicht vollständig in das Chromosom integrierbares Plasmid ist und die genannte DNA-Sequenz in das Gen int integriert ist.

4. Verfahren nach einem der Ansprüche 1 bis 3, dadurch gekennzeichnet, daß die Sequenz att ausgewählt wird aus:
- der Sequenz
TTCTCTGTCGGGGTGGCGGGATTTGAACCCACGACCTCTTCGTCCCGAA und
- der Sequenz, die eine Homologie von mindestens 50 % mit dieser Sequenz aufweist.

5. Verfahren nach Anspruch 4, dadurch gekennzeichnet, daß die Sequenz att ausgewählt wird aus solchen, die sich in S. antibioticus (DSM 40868), S. bikiniensis (ATCC 11062), S. parvulus (ATCC 12434), S. glaucescens (ETH22794), S. actuosus (ATCC25421), S. coelicolor (A3(2)), S. ambofaciens, S. lividans TK64(66) und S. griseofuscus (DSM40191) hybridisieren.

6. Verfahren nach einem der Ansprüche 1 bis 5, dadurch gekennzeichnet, daß die Fragmente att und int aus einem Fragment von pSAM2 stammen, aus dem etwa 600 bp beiderseits der Stelle EcoRI Nr. 33 deletiert worden sind.

7. Verfahren nach Anspruch 6, dadurch gekennzeichnet, daß die Deletion Aufschluß gibt über ein Fragment, das sich zwischen zwei wiederkehrenden Sequenzen erstreckt.

8. Verfahren nach einem der Ansprüche 1 bis 5, dadurch gekennzeichnet, daß er außerdem funktionelle Transfer-Funktionen in dem genannten Stamm enthält.

9. Verfahren nach einem der Ansprüche 1 bis 8, dadurch gekennzeichnet, daß die Sequenz, die für ein vorgegebenes Protein codiert, die Sequenz ist, die für die Resistenz gegenüber Thiostrepton codiert.

10. Actinomyceten-Stamm, wie er bei Durchführung des Verfahrens nach einem der Ansprüche 7 bis 10 erhalten wird.

11. Stamm nach Anspruch 10, dadurch gekennzeichnet, daß es sich dabei um einen Streptomyces-Stamm handelt.

12. Verfahren zur Herstellung eines vorgegebenen Proteins, dadurch gekennzeichnet, daß man einen Stamm nach einem der Ansprüche 11 oder 12 in einem geeigneten Kulturmedium fermentieren läßt und daß man das vorgegebene Protein daraus gewinnt (abtrennt).
